# EUROPÄISCHE PATENTSCHRIFT

(11) **EP 0 562 329 B1**
(45) Veröffentlichungstag und Bekanntmachung des Hinweises auf die Patenterteilung: **19.07.1995**
(21) Anmeldenummer: 93103532.3
(22) Anmeldetag: 05.03.1993
(51) Int. Cl.: C09C 1/00, C09D 5/36, C09D 11/00, C09D 7/12, C08K 3/00, A61K 7/00, C04B 33/14, C09C 1/64

(54) **Als Glanzpigmente geeignete Mischungen**
Glossy pigment mixtures
Mélanges de pigments brillants

(30) Priorität: 21.03.1992 DE 4209242
(43) Veröffentlichungstag der Anmeldung: 29.09.1993
(73) Patentinhaber: BASF Aktiengesellschaft, 67063 Ludwigshafen (DE)
(72) Erfinder: Schmidt, Helmut, W-6522 Osthofen (DE); Ostertag, Werner, Dr., W-6718 Gruenstadt (DE); Mronga, Norbert, Dr., W-6915 Dossenheim (DE); Schmid, Raimund, Dr., W-6730 Neustadt (DE)

(56) Entgegenhaltungen:
- EP-A- 0 033 457
- EP-A- 0 045 851
- FR-A- 2 352 867
- DATABASE WPI, Section Ch, Week 7932, Derwent Publications Ltd., London, GB; Class G01, AN 79-5897B; & JP-A-54 081 337

## Beschreibung

Die vorliegende Erfindung betrifft neue als Glanzpigmente geeignete Mischungen, im Wesentlichen bestehend aus
A) 5 bis 50 Gew.-% mit Eisenoxid beschichtete Aluminiumteilchen und
B) 50 bis 95 Gew.-% mit Eisenoxid beschichteter Glimmerteilchen, die in einer ersten Schicht bereits mit farblosen, hochbrechenden Metalloxiden belegt sein können.

Weiterhin betrifft die Erfindung die Herstellung dieser Mischungen sowie ihre Verwendung als Trockenpigmente in Druck- und Beschichtungsverfahren sowie allgemein zur Einfärbung von Lacken, Druckfarben, Kunststoffen, Zubereitungen der dekorativen Kosmetik und keramischen Produkten.

Glanz- oder Effektpigmente werden in zunehmendem Maße in vielen Bereichen der Technik eingesetzt, beispielsweise in Automobillacken, in der dekorativen Beschichtung, der Kunststoffeinfärbung, in Druck-, Anstrich-, insbesondere Sicherheitsfarben sowie in der Kosmetik.

Ihre optische Wirkung beruht auf der gerichteten Reflexion an überwiegend flächig ausgebildeten, ausgerichteten metallischen oder stark lichtbrechenden Pigmentteilchen. Je nach Art der Pigmentteilchen spricht man auch von Metalleffektpigmenten (z.B. Aluminium, Zink, Kupfer oder deren Legierungen) oder Perlglanzpigmenten (z.B. auf Basis beschichteter Glimmer wie Muskovit, Phlogopit und Biotit, Talkum oder Glas).

Die Glanzpigmente können durch Beschichtung der Ausgangssubstrate mit dünnen Filmen aus hochbrechenden Oxiden wie Chrom(III)oxid, vor allem Eisenoxid und Titanoxid mehrphasig aufgebaut sein. Durch Interferenz und gegebenenfalls Absorption ergibt sich in diesen Fällen in Abhängigkeit von der Dicke der Oxidschicht eine Vielzahl von Farbtonvariationen; man nennt diese Pigmente daher auch Interferenzpigmente.

Durch die gerichtete Reflexion des einfallenden Lichtes an den plättchenförmigen Pigmentteilchen zeigen die z.B. in Lack ausgerichteten beschichteten Glanzpigmente Goniochromatizität, d.h. der Farbeindruck (Helligkeit und/oder Farbton und/oder Farbsättigung) ihrer Lackierung ändert sich in Abhängigkeit vom Belichtungs- bzw. Betrachtungswinkel. Diese Effekte lassen sich auf ein kompliziertes Zusammenspiel von Reflexion und Transmission des auftreffenden Lichts zurückführen, wobei dessen Farbe durch an den Pigmentteilchen hervorgerufene Phänomene wie Interferenz an dünnen Schichten und Absorption an farbigen Zentren verändert werden kann.

Beispiele für derartige Pigmente sind die in der EP-A-33457 bzw. der EP-A-45851 und der US-A-3 087 829 beschriebenen mit Eisenoxid belegten Aluminium- und Glimmerpigmente.

Insbesondere zur Erzielung von brillanten Gelb-, Gold- und Rottönen und, im Gegensatz zu den Glimmerpigmenten, gleichzeitig gutem Deckvermögen sind die aus der EP-A-33457 bekannten, mit Fe₂O₃ belegten Aluminiumpigmente von Interesse.

Nachteilig ist bei diesen Pigmenten jedoch die leichte Entzündlichkeit ihrer Pulver an Luft sowie ihre Staubexplosionsgefährlichkeit, die ihre Anwendung in Form von zum Beispiel mit Schwerbenzin angefeuchteten Pigmentpasten erforderlich machen und allen Anwendungen in Form von Trockenpigmenten entgegenstehen. Dies gilt vor allem für Druck- und Beschichtungsverfahren, beispielsweise für den Bronzierdruck, bei dem zunächst eine nicht pigmentierte Bindemittellösung angedruckt wird und die benetzten Stellen anschließend über das Bronzierwerk mit trockenem Pigment bestäubt werden. Problematisch ist auch die großtechnische Herstellung der mit Fe₂O₃ belegten Aluminiumpigmente bei hohen Eisenoxidgehalten.

Der Erfindung lag daher die Aufgabe zugrunde, die Entzündlichkeit sowie die Staubexplosionsgefährlichkeit von mit Eisenoxid, insbesondere mit Fe₂O₃, belegten Aluminiumpigmenten zu verringern bzw. zu beseitigen.

Demgemäß wurden die neuen Mischungen, die im wesentlichen aus A) 5 bis 50 Gew.-% mit Eisenoxid beschichteter Aluminiumteilchen und B) 50 bis 95 Gew.-% mit Eisenoxid beschichteter Glimmerteilchen, die in einer ersten Schicht bereits mit farblosen hochbrechenden Metalloxiden belegt sein können, bestehen.

Weiterhin wurde ein Verfahren zur Herstellung dieser Mischungen gefunden, welches dadurch gekennzeichnet ist, daß man die Aluminium- und Glimmerteilchen gemeinsam in einer Wirbelschicht durch Gasphasenzersetzung von Eisencarbonylen in Gegenwart von Sauerstoff und gewünschtenfalls Wasserdampf mit Eisenoxid belegt.

Außerdem wurden die Verwendung dieser Mischungen als Trockenpigmente in Druck- und Beschichtungsverfahren sowie ein Verfahren zur Herstellung von bedruckten oder beschichteten Erzeugnissen gefunden, welches dadurch gekennzeichnet ist, daß man auf die noch nicht bedruckten oder beschichteten Erzeugnisse nach den hierfür bekannten Methoden zunächst eine Bindemittelschicht aufbringt und anschließend die Mischungen in Form von Trockenpigmenten aufstäubt oder aufsprüht.

Nicht zuletzt wurde auch die allgemeine Verwendung der Mischungen zur Einfärbung von Lacken, Druckfarben, Kunststoffen, Zubereitungen der dekorativen Kosmetik und keramischen Produkten gefunden.

Die erfindungsgemäßen Mischungen enthalten 5 bis 50 Gew.-% der Komponente A und 50 bis 95 Gew.-% der Komponente B, bevorzugt 15 bis 40 Gew.-% A und 60 bis 85 Gew.-% B sowie besonders bevorzugt 25 bis 35 Gew.-% A und 65 bis 75 Gew.-% B. Selbstverständlich können auch weitere für den jeweiligen Anwendungszweck übliche Zusätze enthalten sein.

Überraschenderweise werden Brandgefährlichkeit und Staubexplosionsgefährlichkeit des mit Fe₂O₃ belegten Aluminiumpigmentes durch die Anwesenheit des Perlglanzpigmentes deutlich herabgesetzt. Die Pigmentgemische sind nicht staubexplosionsgefährlich und nur schwer entzündlich. So kann bei den erfindungsgemäßen Gemischen in Kontakt mit bis zu 500°C heißen Körpern an Luft keine Zündung festgestellt werden, während die Zündtemperatur des reinen Fe₂O₃-belegten Aluminiumpigmentes bei 360°C liegt. Auch ist nach lokaler Einwirkung einer hinreichend starken Zündquelle keine Brandausbreitung zu beobachten, während Pulver aus Fe₂O₃-belegten Aluminiumteilchen unter analogen Bedingungen abbrennen.

Die in den erfindungsgemäßen Mischungen enthaltenen mit Eisenoxid beschichteten Aluminiumpigmente und ihre Herstellung sind in der EP-A-33457 beschrieben.

Bevorzugt sind insbesondere mit Fe₂O₃ belegte Aluminiumpigmente, da mit ihnen vor allem brillante und hochglänzende Gelb-, Gold- und Rottöne zu erhalten sind.

Die als Substrat dienenden plättchenförmigen Aluminiumteilchen können durch Herausstanzen aus Aluminiumfolie oder nach bekannten Verdüsungs- oder Mahlverfahren hergestellt werden und haben in der Regel mittlere Partikeldurchmesser von 10 bis 120 »m. Die Teilchengröße wird vorteilhaft auf den jeweiligen Verwendungszweck abgestimmt. Für den Bronzierdruck sind beispielsweise mittlere Teilchendurchmesser von 10 bis 50 »m bevorzugt, für eine Anwendung in Lacken sollten die mittleren Teilchendurchmesser vorzugsweise von 12 bis 24 »m betragen.

Die belegten Aluminiumpigmente enthalten in der Regel 5 bis 65, vorzugsweise 10 bis 55 Gew.-% Fe₂O₃.

Die erhaltenen Farbtöne hängen in bekannter Weise sowohl von der Größe der Teilchen als auch besonders von der Dicke der Oxidschicht ab. So werden mit zunehmender Beschichtung nacheinander die Farbtöne Blaßgelb, Gelb, Gold, Rot, Violett, Graugelb, Graugold, Rotgold, Kirschrot durchlaufen.

Beispielsweise sind die trockenen Pigmentpulver bei einer spezifischen Oberfläche (BET) von 2,5 m²/g bei einem Fe₂O₃-Gehalt von 16 bis 22 Gew.-% gold (Fe₂O₃-Schichtdicke etwa 25 nm) und von 45 bis 60 Gew.-% kirschrot gefärbt (Schichtdicke etwa 115 nm).

Die in den erfindungsgemäßen Mischungen eingesetzten mit Eisenoxid belegten Glimmerpigmente sowie ihre Herstellung sind aus der EP-A-45851 bekannt.

Auch hier sind insbesondere mit Fe₂O₃ beschichtete Glimmerschuppen bevorzugt, die in der Regel 5 bis 65, vorzugsweise 10 bis 55 Gew.-% Fe₂O₃ enthalten.

Als Substrat kommen insbesondere helle bzw. weiße Glimmer in Betracht, wobei Schuppen von vorzugsweise naß vermahlenem Muskovit besonders bevorzugt sind. Selbstverständlich sind auch andere blättchenförmige Silikatschuppen wie Phlogophit, künstliche Glimmer oder Glasschuppen geeignet. Außerdem können die Glimmerteilchen auch in erster Schicht mit farblosen hochbrechenden Metalloxiden, insbesondere Titan- oder Zirkondioxid, belegt sein. Solche Pigmente sind beispielsweise aus der US-A-3 087 828 bekannt.

Die Glimmerteilchen haben im allgemeinen mittlere Teilchengrößen in der Hauptdimension von 5 bis 400 »m, vorzugsweise von 10 bis 100 »m und insbesondere von 30 bis 70 »m, während ihre mittlere Dicke in der Regel von 0,03 bis 30 »m, bevorzugt von 0,2 bis 5 »m beträgt.

Analog zu den beschichteten Aluminiumpigmenten ist auch der Farbton der beschichteten Glimmerpigmente sowohl von der Korngröße als auch vom Gehalt an Fe₂O₃ abhängig. So sind beispielsweise die trockenen Glimmerpigmente bei einer mittleren Korngröße von 5 bis 50 »m und einer spezifischen Oberfläche (BET) von etwa 3 m²/g bei einem Fe₂O₃-Gehalt von 10 bis 25 Gew.-% gelb bzw. golden (Fe₂O₃-Schichtdicke etwa 25 nm) und von 45 bis 55 Gew.-% rot (Schichtdicke etwa 120 nm).

Beim erfindungsgemäßen Verfahren zur Herstellung der neuen Pigmentmischungen werden Aluminium- und Glimmerteilchen gemeinsam in einer Wirbelschicht durch Gasphasenzersetzung von Eisenpentacarbonyl mit Eisenoxid belegt.

Verfahrenstechnisch geht man dabei zweckmäßigerweise wie folgt vor: Ein Gemisch der Pigmentteilchen im gewünschten Gewichtsverhältnis wird in einem beheizbaren Wirbelbettreaktor, wie er beispielsweise aus der EP-A-33 457 bekannt ist, zunächst mit einem Wirbelgas, das über mehrere im unteren Teil des Reaktors seitlich angebrachte Düsen eingeleitet wird und nicht mehr als 5 Vol.-% Sauerstoff enthalten sollte, also vorzugsweise mit einem entsprechenden Stickstoff/Luft-Gemisch verwirbelt und dann auf eine Temperatur von in der Regel 100 bis 400°C, bevorzugt 180 bis 250°C erhitzt.

Elektrostatische Aufladungen können vorteilhaft vermieden werden, wenn gleichzeitig Wasserdampf in den Reaktor eingeleitet wird. Das kann in einfacher Weise geschehen, indem man das Wirbelgas oder einen Teil des Wirbelgases durch erwärmtes Wasser leitet, wobei es sich mit Wasserdampf belädt, oder über Zuführen des Wasserdampfs über eine zusätzliche seitliche Düse erfolgen.

Nach Erreichen der Reaktionstemperatur wird verdampftes Eisencarbonyl, insbesondere Eisenpentacarbonyl, vorzugsweise im Gemisch mit einem inerten Gas wie Stickstoff oder Argon über eine Düse eingetragen und im Reaktor kontrolliert oxidiert. Um gleichmäßige Beschichtungen der Pigmentteilchen mit Eisenoxid zu erreichen, sollte die Konzentration des Eisenpentacarbonyldampfes, bezogen auf die Gesamtmenge der in den Reaktor eingeleiteten Gase, in der Regel nicht mehr als 5 Vol.-%, vorzugsweise nicht mehr als 1 Vol.-% betragen.

In Abhängigkeit von der Dauer der Behandlung, der Konzentration des gasförmigen Carbonyls und dem Verhältnis von eingeleitetem Eisenpentacarbonyl und vorgelegten Pigmentteilchen gelingt es, Pigmente herzustellen, deren Farbton von hellgoldgelb bis dunkelviolett gezielt und reproduzierbar eingestellt werden kann.

Ist der gewünschte Farbton erreicht, was an einer entnommenen Probe beurteilt werden kann, wird der Reaktor abgekühlt, und das abgekühlte Produkt wird ausgetragen.

Von besonderem Vorteil ist, daß mit Hilfe des erfindungsgemäßen Herstellungsverfahrens auch die Aluminiumpigmente mit einem Fe₂O₃-Gehalt von größer als 20 Gew.-%, insbesondere die interessanten rot gefärbten Pigmente mit einem Fe₂O₃-Gehalt von etwa 45 bis 60 Gew.-% auch in technischem Maßstab problemlos zugänglich sind, da ein Durchreagieren des Reaktorinhalts im Sinne einer Thermitreaktion (2Al + Fe₂O₃ → Al₂O₃ + 2Fe) durch die Anwesenheit der Glimmerteilchen verhindert wird.

Selbstverständlich können die erfindungsgemäßen Mischungen, insbesondere solche mit geringeren Fe₂O₃-Gehalten, auch durch nachträgliches Mischen der bereits beschichteten Komponenten in einem flüssigem Medium wie Ethanol oder Aceton und anschließendes Trocknen oder trocken unter einem Schutzgas wie Stickstoff oder Argon vorzugsweise direkt im Wirbelbett nach der Beschichtung des Aluminiumteilchen erhalten werden. In diesem Fall ist es möglich, durch Mischen unterschiedlich gefärbter Komponenten, beispielsweise von goldenem Aluminiumpigment mit gelbem bis rotviolettem Glimmerpigment, zusätzlich interessante koloristische Effekte zu erzielen.

Die erfindungsgemäßen Mischungen können grundsätzlich in allen für Glanzpigmente üblichen Bereichen der Technik eingesetzt werden. Besonders günstig ist, daß sie auch in trockener Form gefahrlos gehandhabt werden können und damit beispielsweise in Druck- und Beschichtungsverfahren zum Einsatz kommen können, bei denen ein Trockenpigment verwendet wird.

Ein Beispiel für ein derartiges Verfahren ist der Bronzierdruck, bei dem, wie eingangs erwähnt, zunächst nur eine Bindemittellösung angedruckt wird und die benetzten Stellen anschließend mit trockenem Pigment bestäubt werden. Die genaue Vorgehensweise entspricht dabei den allgemein bekannten Techniken. Als Bindemittel kommen die üblicherweise verwendeten Mittel wie Leinölfirnis und phenolmodifizierte Kolophoniumharzester in Betracht.

Die erfindungsgemäßen Mischungen zeichnen sich durch ihre besonders vorteilhafte Koloristik, ihr Deckvermögen, ihre hohe Ergiebigkeit und die Wirtschaftlichkeit ihrer Anwendung aus. Sie sind auch in trockener Form weder brand- noch staubexplosionsgefährlich. Mit ihnen können vor allem brillante Rot- bis Gelb- und Goldtöne erzielt werden. Dabei zeigen insbesondere die in Lack applizierten Rotpigmente neben ihrem intensiven Rotton auch das metallische Glänzen des Aluminiumsubstrats, was sie zu besonders interessanten Glanzpigmenten macht.

### Beispiele

### A. Herstellung von erfindungsgemäßen Mischungen

### A.1 Herstellung durch gemeinsames Beschichten der Aluminium- und Glimmerteilchen

### Beispiel 1

Eine Mischung aus 60 g Aluminium, bestehend aus 30 g Aluminiumpulver (mittlerer Teilchendurchmesser 20 »m, BET-Oberfläche 4,5 m²/g) und 30 g gröberem Aluminiumpulver (mittlerer Teilchendurchmesser 60 »m, BET-Oberfläche 1,5 m²/g) und 140 g gemahlenem Glimmer (mittlerer Teilchendurchmesser < 100 »m) wurde in einem von außen beheizbaren Wirbelbettreaktor aus Glas mit einem Durchmesser von 8 cm und einer Höhe von 80 cm mit Glasfrittenboden und oben eingehängten, mit einem Stickstoff-Jet abreinigenden Filterstrümpfen und einer seitlich oberhalb des Frittenbodens eingebrachten Düse zur Gaseinleitung auf 180°C erhitzt. Insgesamt wurden 1000 l/h Wirbelgas eingeleitet, davon 400 l/h Stickstoff, 400 l/h durch eine auf Raumtemperatur gehaltene Vorlage mit Eisenpentacarbonyl geleiteter Stickstoff und 200 l/h durch eine auf 40°C temperierte Wasservorlage geleitete Luft. Innerhalb von 20 h wurden portionsweise ingesamt 840 g Eisenpentacarbonyl in den Reaktor überführt und als Eisenoxidfilm auf den plättchenförmigen Substraten abgeschieden. Nach dem Abkühlen wurde das Produkt ausgetragen.

Das erhaltene rote Pigment hatte einen Fe₂O₃-Gehalt von 51,5 Gew.-% und zeigte hohen Glanz, hohe Farbstärke und hohes Deckvermögen. Es konnte weder durch Funken noch durch Flammen gezündet werden.

### Beispiel 2

Analog Beispiel 1 wurde eine Mischung aus 60 g Aluminium (mittlerer Teilchendurchmesser 60 »m, BET-Oberfläche 1,5 m²/g) und 140 g des Glimmerpigmentes Flonac®ME 11 (Kemira Oy, Pori, Finnland) mit Fe₂O₃ belegt, wobei innerhalb von 10 h portionsweise 145,3 g Eisenpentacarbonyl zugeführt wurden.

Das erhaltene goldfarbene Pigment hatte einen Fe₂O₃-Gehalt von 12,0 Gew.-% und zeigte starken Glanz.

### Beispiel 3

Analog Beispiel 1, jedoch in einem etwas größer ausgelegten Wirbelbettreaktor wurde eine Mischung aus 260 g Aluminium (mittlerer Teilchendurchmesser 20 »m, BET-Oberfläche 4,5 m²/g) und 600 g gemahlenem Glimmer (mittlerer Teilchendurchmesser < 100 »m) unter Verwirbelung mit insgesamt 2300 l/h Wirbelgas (1500 l/h Stickstoff, 400 l/h mit Eisenpentacarbonyl beladener Stickstoff und 400 l/h mit Wasserdampf beladene Luft) und portionsweiser Zuführung von 1670 g Eisenpentacarbonyl in 30 h mit Eisenoxid belegt.

Das erhaltene goldfarbene Pigment hatte einen Fe₂O₃-Gehalt von 44,3 Gew.-% und zeigte starken Glanz.

### Beispiel 4

Analog Beispiel 3 wurde die Beschichtung mit 1960 g Eisenpentacarbonyl, das in 40 h portionsweise zugeführt wurde, vorgenommen.

Das erhaltene orangerot gefärbte Pigment hatte einen Fe₂O₃-Gehalt von 48,6 Gew.-% und zeigte starken Glanz.

### A.2 Herstellung durch Mischen der bereits beschichteten Aluminium- und Glimmerteilchen

### Beispiel 5

Eine Mischung aus 1 kg eines mit Eisenoxid beschichteten, goldfarbenen Aluminiumpigments (78 Gew.-% Al, 22 Gew.-% Fe₂O₃, Teilchendurchmesser 10 - 48 »m) und 2 kg eines mit Eisenoxid beschichteten, goldfarbenen Glimmerpigments (77 Gew.-% Muskovit, 23 Gew.-% Fe₂O₃, Teilchendurchmesser 10 - 48 »m) wurde unter Stickstoff in einem Rührkessel in 10 l Aceton 10 min gerührt, abfiltriert und bei 105°C getrocknet.

### Beispiel 6

Analog Beispiel 1, jedoch ohne Glimmerzusatz, wurden 40 g mit Eisenoxid beschichtetes, goldfarbenes Aluminiumpigment (88 Gew.-% Al, 12 Gew.-% Fe₂O₃, Teilchendurchmesser 10 - 65 »m) hergestellt. Nach dem Abkühlen wurden 60 g mit Eisenoxid beschichtetes, goldfarbenes Glimmerpigment (75 Gew.-% Muskovit, 2 Gew.-% TiO₂, 23 Gew.-% Fe₂O₃, Teilchendurchmesser 10 - 65 »m) eingetragen. Diese Mischung wurde 15 min mit 350 l Stickstoff verwirbelt und ausgetragen.

### Vergleichsbeispiel V

Analog Beispiel 1 wurden die Aluminiumplättchen ohne Zusatz von Glimmer mit Fe₂O₃ belegt. Das erhaltene goldfarbene Pigment enthielt 22 Gew.-% Fe₂O₃.

### B. Bestimmung der Entzündlichkeit, der Zündtemperatur und der Staubexplosionsgefährlichkeit

Die Entzündlichkeit der Pigmentpulver an Luft wurde nach 84/449/EWG, Anhang A 10 bestimmt. Dabei wurden als Zündquellen eine 2 cm große Methangasflamme, Cereisenfunken und ein Schwarzpulver-Zündstrahl eingesetzt. Gemessen wird die kürzeste Abbrandzeit einer 100 mm langen Schüttung. Leichtentzündlich sind Stoffe, deren kürzeste Abbrandzeiten unter 45 sec liegen.

Zur Bestimmung der Zündtemperatur wurden die Pigmentpulver an der Luft jeweils in Kontakt mit einer 100 bis 800°C heißen Heizplatte gebracht. Die Zündtemperatur ist als die niedrigste Temperatur einer heißen Fläche, an der sich ein Probe/Luft-Gemisch optimaler Zusammensetzung unter festgelegten Bedingungen gerade noch entzündet.

Die Staubexplosionsgefährlichkeit der Pigmentpulver wurde im offenen Hartmannrohr gemessen. Dazu wurden in einem senkrecht stehenden, unten geschlossenen und oben mit einem lose aufliegenden Klappdeckel versehenden Glasrohr (Volumen 1,2 l) Staubproben in Konzentrationen von 30 bis 1000 g/m³ mit Luft aufgewirbelt und mit elektrischen Dauerfunken (Energie von ca. 4 Joule) und einer Drahtglühwendel (ca. 1200 °C) als Zündquellen zu zünden versucht.

Die Ergebnisse der Untersuchungen sind in der folgenden Tabelle zusammengefaßt.

**Tabelle**

| Bsp. | Entzündlichkeit | Zündtemperatur [°C] | Staubexplosionsgefährlichkeit |
|---|---|---|---|
| 5 | schwer | >500 | keine |
| 6 | schwer | >500 | keine |
| V | leicht | 360 | stark |

Die Pigmentmischungen der Beispiele 1 bis 3 zeigen im Gegensatz zu dem nicht gemischten Fe₂O₃-belegten Aluminiumpigment keine Staubexplosionsgefährlichkeit und sind auch nur schwer entzündlich.

### C. Anwendung von erfindungsgemäßen Mischungen

### Beispiel 1C

Auf einer Bogenoffsetmaschine mit angebautem Bronzierdruck-Kanal wurden Papierbögen für Weinetiketten (Marke Chromolux®, Fa. Teraset) zunächst im Offset-Verfahren mit einem nicht pigmentierten Klebefirnis (Bronzierfirnis) aus 95 Gew.-% Leinölfirnis und phenolmodifiziertem Kolophoniumharzester und 5 Gew.-% Polyvinyltoluol bedruckt und dann sofort in die Bronzierstation weitergeführt, wo sie mit der Pigmentmischung aus Beispiel 5 bestäubt wurden. Überschüssiges Pigmentpulver wurde durch eine Samtrakel entfernt.

Die Druckgeschwindigkeit betrug 2600 Bogen/h. Der Verbrauch lag bei nur 0,9 kg Pigment, während von einem konventionellen Metallpigment (goldfarbenes Kupfer/Zink-Pigment) unter analogen Bedingungen 2,7 kg verbraucht wurden.

Die bedruckten Weinetiketten zeigten hochglänzenden goldfarbenen Aufdruck mit hohem Deckvermögen. Ein Verschmutzen der nicht bedruckten Papierstellen durch anhaftendes überschüssiges Pigmentpulver wurde nicht beobachtet.

### Beispiel 2C

Analog Beispiel 1C wurde die Pigmentmischung aus Beispiel 6 zum Bedrucken der Weinetiketten eingesetzt.

Die Druckgeschwindigkeit betrug 2500 Bogen/h, der Verbrauch an Pigment lag bei demselben Motiv wie in Beispiel 1C bei 1,9 kg.

Die bedruckten Weinetiketten zeigten hochglänzenden bleichgoldfarbenen Aufdruck mit hohem Deckvermögen.

### Beispiel 3C

Analog Beispiel 1C wurde die Pigmentmischung aus Beispiel 2 zum Bedrucken der Weinetiketten eingesetzt.

Die Druckgeschwindigkeit betrug 2300 Bogen/h, der Verbrauch lag bei 2,0 kg Pigment.

Die bedruckten Etiketten zeigten hochglänzenden roten Aufdruck mit ausgeprägter Winkelabhängigkeit des Farbtons und der reflektierten Helligkeit.

## Patentansprüche

1. Als Glanzpigmente geeignete Mischungen, im wesentlichen bestehend aus
A) 5 bis 50 Gew.-% mit Eisenoxid beschichteter Aluminiumteilchen und
B) 5 bis 95 Gew.-% mit Eisenoxid beschichteter Glimmerteilchen, die in einer ersten Schicht bereits mit farblosen, hochbrechenden Metalloxiden belegt sein können,

2. Verfahren zur Herstellung der Mischungen gemäß Anspruch 1, dadurch gekennzeichnet, daß man die Aluminium- und Glimmerteilchen gemeinsam in einer Wirbelschicht durch Gasphasenzersetzung von Eisencarbonylen in Gegenwart von Sauerstoff und gewünschtenfalls Wasserdampf mit Eisenoxid belegt.

3. Verfahren zur Herstellung von bedruckten oder beschichteten Erzeugnissen, dadurch gekennzeichnet, daß man auf die noch nicht bedruckten oder beschichteten Erzeugnisse nach den hierfür bekannten Methoden zunächst eine Bindemittelschicht aufbringt und anschließend die Mischungen gemäß Anspruch 1 in Form von Trockenpigmenten aufstäubt oder aufsprüht.

4. Verwendung der Mischungen gemaß Anspruch 1 als Trockenpigmente in Druck- und Beschichtungsverfahren nach den hierfür bekannten Techniken.

5. Verwendung der Mischungen gemäß Anspruch 1 zur Einfärbung von Lacken, Druckfarben, Kunststoffen, Zübereitungen der dekorativen Kosmetik und keramischen Produkten.

## Claims

1. Mixtures useful as luster pigments, consisting essentially of
A) from 5 to 50 % by weight of iron oxide-coated aluminum particles and
B) from 5 to 95 % by weight of iron oxide-coated mica particles with or without a prior coating of a colorless, highly refractive metal oxide.

2. A process for preparing the mixtures of claim 1, which comprises coating the aluminum and mica particles conjointly with iron oxide in a fluidized bed by gas phase decomposition of iron carbonyls in the presence of oxygen and, if desired, water vapor.

3. A process for producing printed or coated articles, which comprises first applying a binder layer to the as yet unprinted or uncoated articles in a conventional manner and subsequently applying the mixtures of claim 1 in the form of dry pigments by dusting or spraying.

4. The use of the mixtures of claim 1 as dry pigments in printing and coating methods by the conventional techniques.

5. The use of the mixtures of claim 1 for coloring paints, printing inks, plastics, decorative cosmetic preparations and ceramic products.

## Revendications

1. Mélanges convenant comme pigments brillants, se composant essentiellement de
A) 5 à 50% en poids de particules d'aluminium revêtues d'oxyde de fer, et de
B) 5 à 95% en poids de particules de mica qui sont revêtues d'oxyde de fer et qui peuvent être déjà enduites, en une première couche, d'oxydes métalliques incolores très réfringents.

2. Procédé de préparation des mélanges selon la revendication 1, caractérisé en ce que l'on enduit en commun d'oxyde de fer les particules d'aluminium et de mica en couche fluidisée par décomposition en phase gazeuse de fer-carbonyles en présence d'oxygène et au besoin de vapeur d'eau.

3. Procédé de fabrication de produits imprimés ou enduits, caractérisé en ce que l'on commence par appliquer une couche de liant sur les produits non encore imprimés ou enduits, par des procédés connus à cette fin, puis on applique par saupoudrage ou pulvérisation les mélanges selon la revendication 1 sous forme de pigments secs.

4. Utilisation des mélanges selon la revendication 1 comme pigments secs dans des procédés d'impression et d'enduction suivant des techniques connues à cet effet.

5. Utilisation des mélanges selon la revendication 1 pour la coloration de vernis, d'encres d'imprimerie, de matières plastiques, de préparations utilisées en cosmétique décorative et de produits céramiques.
